# EUROPEAN PATENT APPLICATION

(11) **EP 4 403 841 A1**
(43) Date of publication of application: **24.07.2024**
(21) Application number: 21957395.3
(22) Date of filing: 14.09.2021
(51) Int. Cl.: F24F 8/167

(54) **AIR AND SURFACE PURIFYING DEVICE**

(71) Applicant: Human Wellness Solutions SL, 29004 Málaga (ES)
(72) Inventor: FERNÁNDEZ ROMERO-NIEVA, Pablo Antonio, 29004 Malaga (ES)
(74) Representative: Torner, Juncosa I Associats, SL
(86) International application number: PCT/ES2021/070658
(87) International publication number: WO 2023/041811

(57) **Abstract**

The invention relates to a purifying device (1) that comprises: a pair of substrate panels placed one in front of the other and comprising a plurality of openings for air to pass through, wherein at least one inner face of said panels and one surface of said openings are coated with a photocatalytic material; an ultraviolet emitter that extends between the substrate panels, such that the ultraviolet light emitted is incident on the inner face of the substrate panels and on the surface of the openings of the substrate panels; and a frame to which the substrate panels and the ultraviolet emitter are fixed. Thus, when the airflow passes through the openings of the substrate panels, water condenses on the photocatalytic material and said water reacts in the presence of ultraviolet light to generate hydrogen peroxide molecules.

## Description

### OBJECT OF THE INVENTION

The present invention belongs to the field of the removal of pathogens and other harmful particles from air and surfaces.

The object of the present invention relates to a novel air purifier based on the generation of hydrogen peroxide.

### BACKGROUND OF THE INVENTION

High efficiency particulate air (HEPA) filtration systems are conventional systems for the treatment of ambient air and removal of suspended particles. Although HEPA filtration systems can be useful to remove air particles, they present limitations common to all filtration systems such as, for example, those relating to clogged filters. Furthermore, filtration-based systems are not capable of deactivating chemicals, removing unwanted gases, or removing smaller odor-causing molecules. Another significant drawback of systems of this type is that they require conducting air to the device in order to pass it through the filter, such that not only do they need a long time for cleaning the air in a room, but they also do not remove surface particles.

Other systems can use activated carbon filters or electrostatic filters. Although these systems can improve pollutant retention capacity and filtration efficiency, they still present the aforementioned limitations which are common to filtration-based systems such as filter replacement, degradation of filter performance over time, and incapacity for surface treatment.

Devices commonly referred to as "*ionizers*", which are designed to emit negative ions into surrounding air, are also known. These ions adhere to positively charged pollutants such as pollen and dust, making them heavier and increasing the likelihood of them settling or being more readily retained on a collection plate. However, since many pollutants simply fall to the ground or adhere to walls instead of being removed, they can return to the air upon the dissipation or association of the negative ions. Furthermore, if a collection plate is used, it must be cleaned or replaced frequently as with any filtration system.

Air purification systems which use ultraviolet (UV) radiation to inactivate and/or degrade airborne pollutants are also known. To that end, the air is conducted in order to pass it through the device next to one or more ultraviolet lamps, such that the ultraviolet light disinfects the air before being emitted back into the environment. However, these systems have the drawback that the destruction of many bacteria and pollutants, particularly spores, requires prolonged exposure time to the ultraviolet light. Furthermore, some volatile organic compounds may also be resistant to ultraviolet energy or, worse still, reactive to the ultraviolet light in such a way that makes them more harmful or exposed to people in the vicinity.

Photocatalytic oxidation (PCO) air purifiers also use ultraviolet light. However, instead of using ultraviolet light to interact directly with passing pollutants, PCO systems focus ultraviolet light on a catalyst material. Water molecules in the ambient air interact with the ultraviolet light and the catalyst, for example titanium dioxide, to generate a variety of oxidants, such as hydroxyl (OH) radicals. The oxidants emitted into the air attack the organic molecule pollutants and degrade same into less harmful substances. Therefore, instead of retaining pollutants, PCO systems are capable of destroying and eliminating pollutants from the treated environment.

However, conventional PCO systems have several limitations. Hydroxyl radicals are only effective for removing pathogens in the proximity of the device since their high reactivity causes them to disappear in milliseconds. Therefore, systems of this type also require passing the air through the device. As a result, the time required for completely disinfecting a room can be between 15 and 20 minutes. Furthermore, since hydroxyl radicals last for a short time, systems of this type cannot remove pathogens deposited on surfaces.

### DESCRIPTION OF THE INVENTION

The present invention solves the preceding problems as a result of an air and surface purifying device capable of generating a small amount of hydrogen peroxide which is emitted into the environment. Hydrogen peroxide lasts considerably longer than hydroxyl radicals and is therefore capable of purifying both the air and the surfaces of the room being disinfected. A much higher disinfection level is thereby achieved compared to conventional PCO systems.

An additional advantage of the present invention is that, due to the wavelengths of the ultraviolet light emitted, ozone is not produced. The absence of ozone is advantageous because it is known that ozone can be harmful to people's health due to respiratory tract irritation. In contrast, hydrogen peroxide at low concentrations is harmless for human beings. Therefore, the device of the present invention can be used when people are in the room without causing any harm.

The present invention describes an air and surface purifying device fundamentally comprising a pair of substrate panels, an ultraviolet emitter, and a frame to which the substrate panels and the ultraviolet emitter are fixed. Each of these elements is defined in greater detail below.

### a) Substrate panels

The term refers to a pair of substrate panels, each of which comprises a plurality of openings for air to pass through same. The substrate panels are arranged in parallel one in front of the other such that a space between both forms an inner cavity. Furthermore, at least one inner face of said substrate panels and one inner surface of said openings of the substrate panels are coated with a photocatalytic material. In this context, it is understood that the inner face of the substrate panels is that face oriented towards the inside of the cavity, i.e., towards the other substrate, whereas the inner surface of the openings are surfaces having a normal contained in a plane parallel to the plane of the plate itself. In other words, they are inner surfaces of each of the individual holes.

The panels will usually have an essentially rectangular shape the thickness of which can range, for example, between about 5 mm and 30 mm. The panels are made of aluminum, which ensures lightweightness while at the same time allowing impregnation with the required photocatalytic material layers. The openings are designed such that they allow air to pass through the panels with the lowest possible head loss so as not to slow down the airflow excessively. At the same time, the openings have a configuration which maximizes the air contact surface to cause water to condense on said surface as much as possible. The reason is that the greater the amount of water condensed on the surface of the holes and the inner face of the panels the higher the amount of hydrogen peroxide generated will be.

According to a particularly preferred embodiment of the invention, the substrate panels have a honeycomb shape, wherein the openings are hexagonal. It has been verified that this shape is optimal for minimizing shading and obtaining the largest amount of area on which to perform the photocatalytic process, thereby helping to maximize water absorption and subsequent oxidation due to the larger amount of irradiated area per material surface.

The dimension of each hexagonal opening of the honeycomb-shaped substrate panels is also important, since a size that is too large causes a reduction in the air contact surface, whereas a size that is too small causes a larger shaded surface, thereby negatively affecting the efficiency of the material. After various studies, it has been verified that the best results are obtained with an opening size of between 2 millimeters and 3 millimeters, i.e., the diameter of the circle circumscribing each hexagonal opening has a diameter of between 2 millimeters and 3 millimeters.

In terms of the photocatalytic material coating, it is preferably arranged on those surfaces of the substrate panels where ultraviolet radiation strikes, specifically the surface of the inner face of the substrate panels and the inner surface of the openings. The photocatalytic material can be any material as long as it promotes the oxidation reaction of the water condensed on said surfaces to give rise to hydrogen peroxide.

According to a preferred embodiment of the invention, the photocatalytic material is impregnated by layers on the substrate panels, where each layer has a different composition according to its function in the oxidation process of the water and its conversion to hydrogen peroxide. More preferably, the photocatalytic material coating comprises an inner layer comprising titanium dioxide mixed with silver, rhodium, and copper, an intermediate layer comprising manganese and silicon dioxide, and an outer layer comprising hydrophilic materials to facilitate capturing water from the air. Even more preferably, when manufacturing the outer substrate layer, a special drying phase is performed to achieve a medium roughness which facilitates water absorption and allows UV light irradiation at angles of up to 160 degrees, which allows maximum light penetration into the substrate to generate a larger amount of hydrogen peroxide.

Preferably, there is arranged between the layers an interlayer mixing region where the composition is mixed in different percentages to facilitate the chemical reactions on the substrate and to enable UV light waves to be reflected within the substrate as they impact against the metals forming same at different frequencies. In other words, between the inner layer and the intermediate layer, and between the intermediate layer and the outer layer, there are respective mixing regions where the compositions of the corresponding layers are mixed. This is achieved by means of a drying process particularly designed to that end.

### b) Ultraviolet emitter

The term refers to an ultraviolet emitter that extends parallel to the pair of substrate panels. Therefore, the ultraviolet light emitted by the ultraviolet emitter is incident on the inner face of the substrate panels and on the surface of the openings of the substrate panels.

The combination of water condensed on said surfaces with the photocatalytic material on which it can be found and with the ultraviolet radiation emitted by the emitter results in the generation of hydrogen peroxide due to the oxidation of the water. Furthermore, a suitable selection of the frequency of the emitted ultraviolet radiation allows reducing or completely eliminating the generation of ozone, preventing the drawbacks that this entails.

In particular, according to a preferred embodiment of the invention, the ultraviolet light emitted by the ultraviolet emitter has a frequency of between 254 nm and 310 nm. This frequency range causes the excitation of the photocatalytic material to cause the oxidation reaction of the water molecules and, at the same time, intentionally prevents frequencies close to 185 nm to ensure that ozone is not produced.

In principle, the ultraviolet emitter can be of any type as long as it is capable of emitting ultraviolet light that is in the mentioned frequency and that, furthermore, reaches the largest possible part of the mentioned surface of the substrate panels. By way of example, an ultraviolet lamp in the form of a U-shaped tube which extends along the cavity formed between the substrate panels can be mentioned. Alternatively, it would be possible to use an ultraviolet emitter based on a plurality of suitably configured UV LEDs, for example also forming an elongated structure essentially spanning the entire length of the inner cavity.

According to a preferred embodiment of the invention, the ultraviolet emitter is arranged inside the inner cavity. Alternatively, the emitter can be arranged outside the inner cavity next to an outer face of one of the substrate panels.

### c) Frame

This term refers to a frame to which the substrate panels and the ultraviolet emitter are fixed. In other words, the frame constitutes a structure which supports the rest of the elements forming the device, which can furthermore include an electronic control board, as well as other electrical or electronic elements necessary for the operation thereof.

The frame comprises two U-shaped frameworks which retain the photocatalytic substrate panels, the two frameworks being fixed to one another by means of an end plate. Additionally, in the case of using a plurality of UV LEDs, the frame comprises a third framework which retains the UV LED board. This way ensures the creation of turbulence in the airflow which reduces airflow speed inside the holes, and therefore promotes the capturing of more water vapor for subsequent oxidation. Furthermore, the U shape of the frameworks allows part of the ultraviolet light to exit through the sides of the device, such that the heat generated by said light is dissipated to prevent heating which would occur with a closed frame. This heating would not only be dangerous due to the risk of fire, but it would furthermore deteriorate the oxidation process by increasing the internal temperature of the device if the frame were closed.

As a result of this configuration, when the airflow passes through the openings of the substrate panels, water condenses on the photocatalytic material. Said water reacts in the presence of ultraviolet light to generate hydrogen peroxide molecules. The airflow going through the device, for example driven by external fans or blowers when they are installed in a conventional air conditioning system, causes the hydrogen peroxide molecules to be expelled into the environment of the enclosure to be disinfected. Hydrogen peroxide molecules have a lifespan long enough to travel through the enclosure until they hit against walls and other surfaces, thereby cleaning both the air of the enclosure and the surfaces without being harmful for human beings.

### BRIEF DESCRIPTION OF THE DRAWINGS

Fig. 1 shows a perspective view of a first example of the device according to the present invention in an assembled state.
Fig. 2 shows a perspective view of the first example of the device of the present invention in a disassembled state.
Fig. 3 shows a schematic view of the openings of the device of the present invention.
Fig. 4 shows a cross-section of the example of the device according to the present invention.
Fig. 5 shows a perspective view of a second example of the device according to the present invention in an assembled state.
Fig. 6 shows a perspective view of the second example of the device of the present invention in a disassembled state.

### PREFERRED EMBODIMENT OF THE INVENTION

Examples of the device (1) according to the present invention in which the parts forming same can be seen are described below.

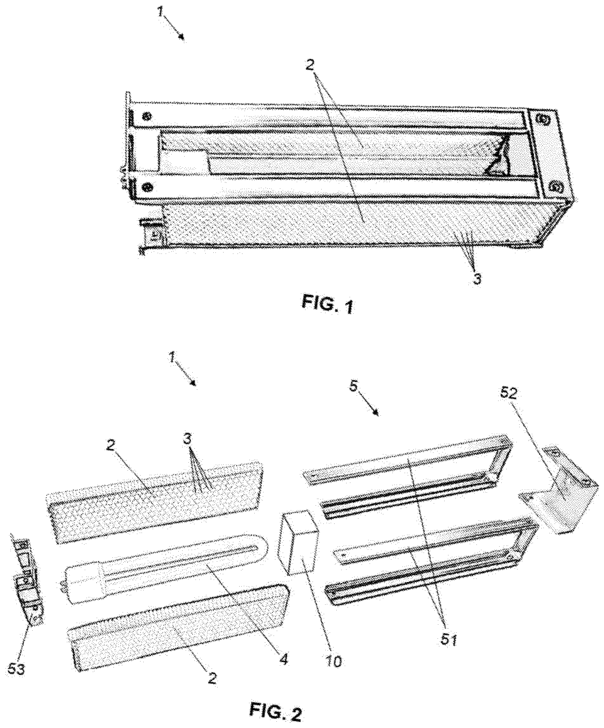

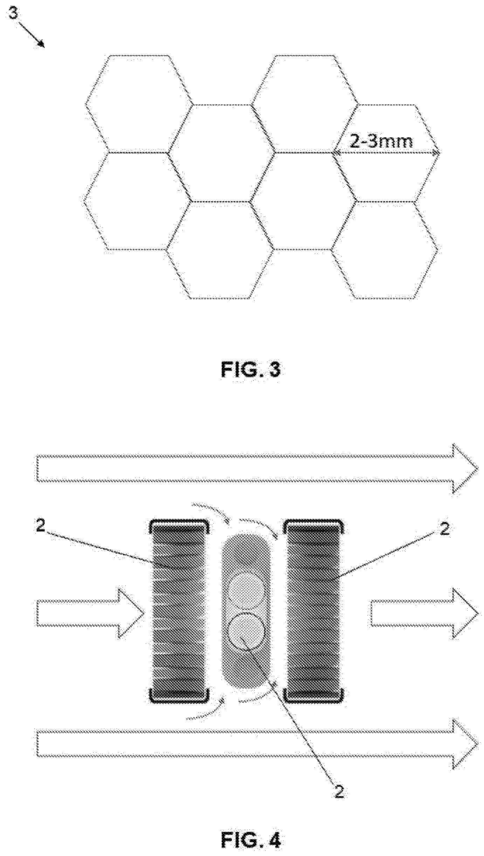

Figs. 1 and 2 show a first example of the device (1) according to the present invention. As can be seen, the device (1) comprises two substrate panels (2) arranged in parallel and provided with a plurality of openings (3) oriented perpendicular to the plane containing each substrate panel (2). The openings (3), as shown in greater detail in Fig. 3, have a hexagonal shape with a dimension between opposite corners that is between 2 mm and 3 mm, whereas the thickness of the substrate panels (2) can be, for example, about 10 mm. A frame (5) is used to fix the two substrate panels (2), said frame comprising two U-shaped frameworks (51) in the cavity of which the substrate panels (2) are fixed, such that they are arranged parallel to one another separated by a distance of several centimeters, for example between 5 centimeters and 10 centimeters. The two U-shaped frameworks (51) are fixed to one another at the base thereof by means of an end plate (52) and at the free ends thereof by means of a stiffening plate (53). The stiffening plate (53) furthermore has means for fixing the base of a U-shaped UV lamp (4), such that the plane of said UV lamp (4) is parallel to the substrate panels (2). The assembled device (1) therefore has the two parallel panels (2) separated by a distance of a few centimeters and the UV lamp (4) is also arranged in parallel, at the midpoint of said distance between the substrate panels (2).

Moreover, although not shown in the figures, the inner faces of the two substrate panels (2) and the inner surfaces of the openings (3) are coated with several layers of photocatalytic material. In particular, there are three layers, wherein the inner layer comprises titanium dioxide mixed with silver, rhodium, and copper, the intermediate layer comprises manganese and silicon dioxide, and the outer layer comprises hydrophilic materials to facilitate capturing water from the air. This photocatalytic coating is selected such that, when the water that condensed on the photocatalytic coating is illuminated with the ultraviolet light emitted by the UV lamp (4), the condensed water is oxidized, causing the generation of hydrogen peroxide.

Thus, this configuration ensures that the ultraviolet light emitted by the UV lamp (4) reaches the largest possible part of the surface coated with the material, generating hydrogen peroxide which disinfects and cleans both surrounding air and surfaces. Furthermore, the frequency of the ultraviolet light emitted by the lamp (4), of between 254 and 310 nm, ensures that ozone is never generated, so this device (1) is compatible with the presence of people in the room.

Fig. 4 schematically shows the operation of this device (1). Airflow goes through the device (1) and causes water to condense on the inner surfaces of the openings (3) of the substrate panels (2). As can be seen, air enters not only through the openings (3) but, since the cavity between both substrate panels (2) is open, also through the space above and below said cavity. This causes the generation of turbulence which improves the performance of the device (1). The combination of water condensed on said surfaces, the UV radiation emitted by the UV lamp (4), and the photocatalytic material coating the inner surfaces of the openings (3) and the inner face of the substrate panels (2), causes the generation of hydrogen peroxide which is entrained by the air stream itself to the enclosure to be disinfected.

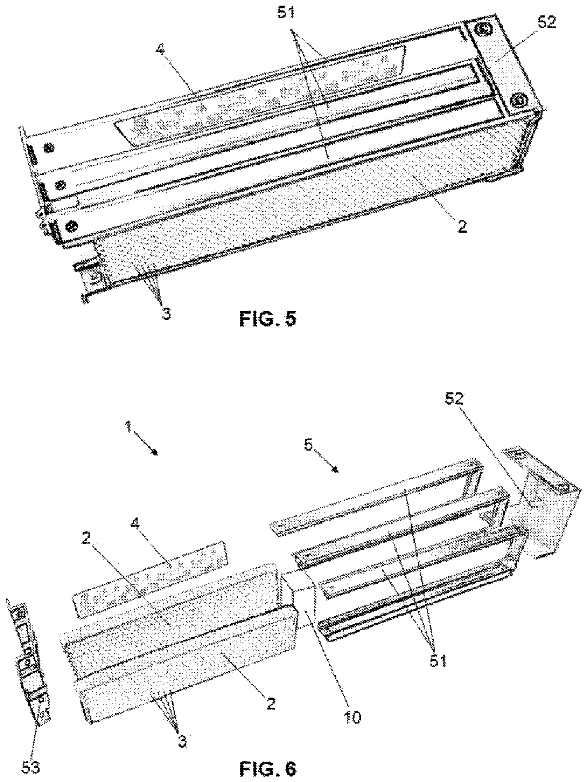

Figs. 5 and 6 show a second example of the device (1) according to the invention in which a plurality of UV LEDs (4) is used instead of the UV lamp used in the preceding example. In this case, as can be seen, the board which contains the UV LEDs (4) is not arranged inside the cavity between the two substrate panels (2), but arranged in parallel next to an outer face of one of said substrate panels (2), i.e., a face opposite that which is oriented towards the cavity. The frame (5) in this case comprises three frameworks (51), two for the respective substrate panels (2) and one for the board which contains the UV LEDs (4). The rest of the operation and elements making up the device (1) of this second example is equivalent to that described above: the UV light emitted by the UV LEDs (4) illuminates the surfaces of the substrate panels (2) and the openings (3) coated with the photocatalytic material, and hydrogen peroxide is generated as a result.

## Claims

1. An air and surface purifying device (1), **characterized in that** it comprises:
- a pair of substrate panels (2), wherein each substrate panel (2) comprises a plurality of openings (3) for air to pass through same, said substrate panels (2) being arranged in parallel one in front of the other such that a space between both forms an inner cavity, and wherein at least one inner face of said substrate panels (2) and an inner surface of said openings (3) of the substrate panels (2) are coated with a photocatalytic material;
- an ultraviolet emitter (4) that extends parallel to the pair of substrate panels (2), such that the ultraviolet light emitted by the ultraviolet emitter (4) is incident on the inner face of the substrate panels (2) and on the inner surface of the openings (3) of the substrate panels (2);
- a frame (5) to which the substrate panels (2) and the ultraviolet emitter (4) are fixed,
such that when the airflow passes through the openings () of the substrate panels (), water condenses on the photocatalytic material and said water reacts in the presence of ultraviolet light to generate hydrogen peroxide molecules.

2. The device (1) according to claim 1, wherein the substrate panels (2) have a honeycomb shape, wherein the openings (3) are hexagonal.

3. The device (1) according to claim 2, wherein the openings (3) have a size of between 2 mm and 3 mm.

4. The device (1) according to any of the preceding claims, wherein the photocatalytic material coating is impregnated by layers on the substrate panels (2).

5. The device (1) according to claim 4, wherein the photocatalytic material coating comprises an inner layer comprising titanium dioxide mixed with silver, rhodium, and copper, an intermediate layer comprising manganese and silicon dioxide, and an outer layer comprising hydrophilic materials to facilitate capturing water from the air.

6. The device (1) according to any of the preceding claims, wherein the ultraviolet emitter (4) is arranged between the two substrate panels (2) inside the inner cavity.

7. The device (1) according to claim 6, wherein the ultraviolet emitter (4) comprises a U-shaped tube lamp that extends parallel to the substrate panels (2).

8. The device (1) according to any of claims 1-5, wherein the ultraviolet emitter (4) is arranged outside the inner cavity next to an outer face of one of the substrate panels (2).

9. The device (1) according to claim 8, wherein the ultraviolet emitter (4) comprises a board comprising a plurality of UV LEDs arranged parallel to the substrate panels (2).

10. The device (1) according to any of the preceding claims, wherein the ultraviolet light emitted by the ultraviolet emitter (4) has a frequency of between 254 nm and 310 nm

11. The device (1) according to any of the preceding claims, wherein the frame (5) comprises U-shaped frameworks (51) which retain the substrate panels (2), the two frameworks being fixed to one another by means of an end plate (52).
